# EUROPEAN PATENT APPLICATION

(11) **EP 3 181 124 A1**
(43) Date of publication of application: **21.06.2017**
(21) Application number: 15003583.0
(22) Date of filing: 16.12.2015
(51) Int. Cl.: A61K 9/20, A61K 31/00

(54) **ABUSE DETERRENT PHARMACEUTICAL DOSAGE FORMS**

(71) Applicant: Universität Basel, 4003 Basel (CH)
(72) Inventor: Huwyler, Jörg, 4144 Arlesheim (CH); Puchkov, Maxim, 4148 Pfeffingen (CH)

(57) **Abstract**

The present invention relates to solid oral pharmaceutical dosage forms that provide extended release of active ingredients and have abuse deterrent properties and the methods of making the same. More particularly, the present invention relates to solid oral extended release abuse deterrent dosage forms comprising at least one polycaprolactone (PCL) and at least one gelling agent.

## Description

### FIELD OF THE INVENTION

The present invention relates to solid oral pharmaceutical dosage forms that provide extended release of active ingredients and have abuse deterrent properties. More particularly, the present invention relates to solid oral extended release abuse deterrent dosage forms comprising polycaprolactone (PCL) and at least one gelling agent and the methods of making the same.

### BACKGROUND OF THE INVENTION

It is known that certain pharmaceutical active ingredients have abuse potential. For example opiates as severe painkillers are known to be used by abusers. Several routes of administration are commonly attempted by abusers. For example, the pharmaceutical dosage form may be chewed, or it may be crushed or pulverized into a powder and administered intranasally. Alternatively, the intact or tampered pharmaceutical dosage form may be dissolved in a suitable solvent and administered parenterally, or the intact or tampered dosage form may be smoked.

Several ways to deter the abuse of the pharmaceutical dosage forms with extended drug release have been developed. First, a chemical approach has been utilized to include an opioid antagonist in opioid pharmaceutical dosage forms. The opioid antagonist, which is not orally active, will substantially block the analgesic effects of the opioid when one attempts to abuse the tampered dosage form via snorting or injecting. Second, averslve agents and/or bitter agents have been added to pharmaceutical formulations to prevent abuse of the active pharmaceutical ingredient. Third, a safer alternative is to incorporate excipients that provide a physical barrier in which abuse of the active pharmaceutical ingredient (API) is deterred. In one case, this is accomplished by incorporating the API into a polymeric matrix tablet containing high molecular weight gel forming polymers such as polyethylene oxide. The polymeric matrix tablet has increased hardness and retains a plastic-like nature after curing at a temperature above the softening temperature of the polyethylene oxide. The resultant tablet dosage form is difficult to crush or chew and forms a viscous gel when the dosage form comes into contact with a suitable solvent.

U.S. 7,776,314 (Grunenthal, GmbH) is directed to a solid administration form, protected from parenteral abuse and containing at least one viscosity-increasing agent in addition to one or more active substances that have parenteral abuse potential.

U.S. 8,114,383 (Grunenthal, GmbH) is directed to a thermoformed dosage form comprising one or more active ingredients with abuse potential selected from the group consisting of opiates and opioids and at least 30 % by weight of polyalkylene oxide having a molecular weight of 1-15 million g/mol. The dosage form may also comprise auxiliary substances like viscosity-increasing agents.

WO 2010/032128 (Purdue Pharma L.P.) is directed to solid oral extended release pharmaceutical dosage forms comprising a melt formed multi particulate extended release matrix formulation, comprising poly(ε-caprolactone).

WO 2014/011830 (Mallinckrodt LLC) is directed to a pharmaceutical composition comprising at least one hydrophilic plastomer, optionally at least one hydrophilic elastomer, and at least one deliquescent plasticizer.

However, there is continuing need for improved pharmaceutical oral dosage forms that provide extended release of the API and are resistant to abuse. Additionally, there is a need for an easy manufacture of said dosage forms without utilizing complicated processes, like (hot) melt extrusion processes.

### SUMMARY OF THE INVENTION

The object of the present Invention is to provide improved abuse deterrent oral extended release pharmaceutical dosage forms and methods for making the same. This object is solved by the subject-matter of the independent claims. Preferred embodiments are indicated in the dependent claims.

The extended release abuse deterrent dosage forms according to the present invention comprise at least one polycaprolactone as a matrix polymer. The dosage forms have a breaking strength of at least 500 N.

It has been surprisingly found that an abuse deterrent oral dosage form with improved hardness, gelling and anti-dose dumping properties can be obtained by combining at least one polycaprolactone with at least one gelling agent and optionally at least one alkalizing agent.

The Applicant has identified that when at least one polycaprolactone, at least one gelling agent and optionally at least one alkalizing agent are incorporated into a dosage form, the abuse deterrent properties (hardness, gelling and anti-dose dumping) are improved without inhibiting the releasing of the API.

### DESCRIPTION OF FIGURES

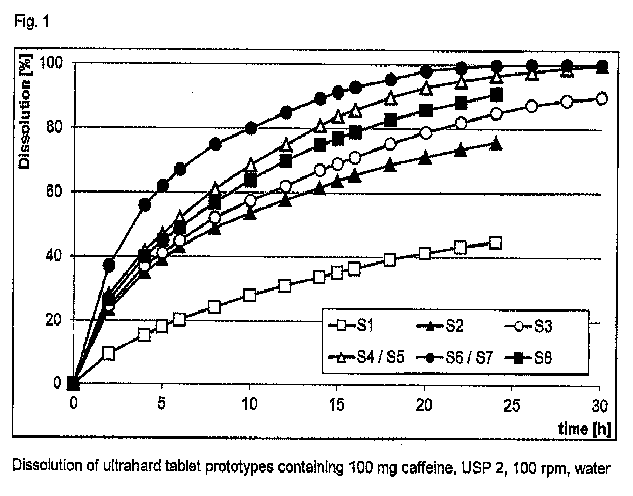

Figure 1 shows the dissolution profiles of the active ingredient from the solid dosage forms with different compositions (Table 1).
Figure 2 shows the dissolution profiles of cut tablets in different solvents.

### DETAILED DESCRIPTION OF THE INVENTION

Hereinafter, the best mode for carrying out the present invention is described in detail.

FDA updated recently the draft guidance for industry related to formulations having abuse deterrent properties. Guidance for Industry: Abuse Deterrent Opioids - Evaluation and Labeling, U.S. Department of Health and Human Services, FDA, CDER, April 2015, the entire contents of which are incorporated herein by reference.

FDA guidance describes the following abuse deterrent formulation categories:
Physical barriers can prevent chewing, crushing, cutting, grating, or grinding of the dosage form. Chemical barriers, such as gelling agents, can resist extraction of the opioid using common solvents like water, simulated biological media, alcohol, or other organic solvents. Physical and chemical barriers can limit drug release following mechanical manipulation, or change the physical form of a drug, rendering it less amenable to abuse.

Agonist/Antagonist combinations - An opioid antagonist can be added to interfere with, reduce, or defeat the euphoria associated with abuse. The antagonist can be sequestered and released only upon manipulation of the product. For example, a drug product may be formulated such that the substance that acts as an antagonist is not clinically active when the product is swallowed but becomes active if the product is crushed and injected or snorted.

Aversion - Substances can be added to the product to produce an unpleasant effect if the dosage form is manipulated or is used at a higher dosage than directed. For example, the formulation can include a substance irritating to the nasal mucosa if ground and snorted.

Delivery System (including use of depot injectable formulations and implants) - Certain drug release designs or the method of drug delivery can offer resistance to abuse. For example, sustained-release depot injectable formulation or a subcutaneous implant may be more difficult to manipulate.

New molecular entities and prodrugs - The properties of a new molecular entity (NME) or prodrug could include the need for enzymatic activation, different receptor binding profiles, slower penetration into the central nervous system, or other novel effects. Prodrugs with abuse-deterrent properties could provide a chemical barrier to the in vitro conversion to the parent opiold, which may deter the abuse of the parent opioid. New molecular entities and prodrugs are subject to evaluation of abuse potential for purposes of the Controlled Substances Act (CSA).

Combination - Two or more of the above methods can be combined to deter abuse.

Novel approaches - This category encompasses novel approaches or technologies that are not captured in the previous categories.

An opioid analgesic submitted for abuse deterrent formulation (ADF) labeling must fulfill the requirements of one or more of these categories.

The object of the present invention is to provide abuse deterrent oral extended release pharmaceutical dosage forms and the methods for making the same which fulfill the above cited FDA requirements.

The pharmaceutical dosage forms disclosed herein comprise at least one polycaprolactone as a matrix former. The Applicants of the present invention have surprisingly found that the combination of the API, at least one polycaprolactone, at least one gelling agent and optionally at least one alkalizing agent results in a composition with optimal hardness (at least 500 N) such that it resists being crushed or ground into powder, Moreover, the resultant composition comprises optimal gelling properties which prevents the parenteral abuse. The dosage form when exposed to a small volume (10 ml or less) of solvent (e.g. water, ethanol, methanol) forms a non-injectable viscous mass, which prevents the uptake by using a syringe.

One aspect of the present disclosure provides extended release, abuse deterrent pharmaceutical dosage forms.

The pharmaceutical dosage form disclosed herein comprises at least one API, at least one polycaprolactone, at least one gelling agent and optionally at least one alkalizing agent.

The pharmaceutical dosage form disclosed herein comprises at least one API (A) or a salt thereof. Suitable APIs Include, without to be limited, opioid analgesic agents, like adulmine, alfentanil, allocryptopine, allylprodine, alphaprodine, anileridine, aporphine, benzylmorphine, berberine, bicuruline, bicucine, bezitramide, buprenorphine, bulbocaprine, butorphanol, clonitazene, codeine, desomorphine, dextromoramide, dezocine, diampromide, diamorphone, dihydrocodeine, dihydromorphlne, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphan, lofentanll, meperidine, meptazinol, metazocine, methadone, metopon, morphine, myrophine, narceine, nicomorphine, norlevorphanol, normathadone, nalorphine, nalbuphene, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tapentadol, tilidine, and tramadol; non-opioid analgesic agents; anti-inflammatory and antirheumatic agents; anesthetic agents; antimigraine agents; antiepileptic agents; anticholinergic agents; anticonvulsant agents; dopaminergic agents; antipsychotic agents; anxiolytic agents; hypnotic and sedative agents; antidepressant agents, anti-dementia agents; anti-adiposity agents; laxative agents and other nervous system agents and combinations thereof.

Any of the above-mentioned APIs may be incorporated In the pharmaceutical dosage form described herein in any suitable form, such as, for example, as a pharmaceutically acceptable salt, uncharged or charged molecule, molecular complex, solvate or hydrate, prodrug, and, if relevant, isomer, enantiomer, racemic mixture, and/or mixtures thereof. Furthermore, the API may be in any of its crystalline, semi-crystalline, amorphous, and in any polymorphic forms.

In certain embodiments, the at least one API in the pharmaceutical dosage form may be an opioid analgesic. Exemplary opioid analgesics include oxycodone, oxymorphone, hydrocodone, hydromorphone, codeine, and morphine. In an exemplary embodiment, the API may be oxycodone hydrochloride. In another exemplary embodiment, the API may be oxymorphone hydrochloride.

The amount of the at least one API may range from about 5 % to about 80 % by weight of the pharmaceutical dosage form. In certain embodiments, the amount of the at least one API may range from about 10 % to about 70 % by weight, and again in certain embodiments, the amount of the at least one API may range from about 10 % to about 50 % by weight of the pharmaceutical dosage form. In certain embodiments, the amount of the at least one API may range from about 15 % to about 30 % by weight of the pharmaceutical dosage form.

The amount of the at least one API in the pharmaceutical dosage form may vary depending upon the active agent. In embodiments in which the API is an opioid analgesic, the amount of opioid in the pharmaceutical dosage form may range from about 2 mg to about 250 mg.

The pharmaceutical dosage form according to the present invention comprises at least one polycaprolactone (B). Polycaprolactone imparts hardness to a solid dosage form matrix when cured. In certain embodiments, the pharmaceutical dosage form according to the present invention may comprise several polycaprolactones having different average molecular weights.

Polycaprolactone (PCL) is a biodegradable polyester produced on the basis of petroleum. It consists of a sequence of methylene units, between which ester groups are formed. Through this very simple structure a slightly limited rotation of the individual chain segments is possible, leading to a very low glass transition point (glass transition temperature, melting temperature; sintering temperature). At room temperature short-chained, amorphous polycaprolactone is correspondingly soft and rubberlike. Because of the uniform structure, however, it is easily crystallized, resulting in the strengthening of the material. Crystalline polycaprolactone resembles polyethylene in crystal structure.

In certain embodiments, the polycaprolactone has a melting point of about 55°C to about 65°C and has an average molecular weight of about 10 000 g/mol to about 250 000 g/mol, preferably of about 20 000 g/mol to about 150 000 g/mol.

The amount of the at least one polycaprolactone may range from about 5 % to about 70 % by weight of the pharmaceutical dosage form. In certain embodiments, the amount of the at least one polycaprolactone may range from about 10 % to about 60 % by weight and again in certain embodiments, the amount of the at least one polycaprolactone may range from about 20 % to about 50 % by weight of the pharmaceutical dosage form. In certain embodiments, the amount of the at least one polycaprolactone may range from about 30 % to about 40 % by weight of the pharmaceutical dosage form.

A gelling agent (C) may facilitate the gelling kinetics of the pharmaceutical dosage form when it is in contact with a small volume of a suitable solvent, without affecting the extended release properties of said dosage form. Non-limiting examples of suitable gelling agents include cellulose, alkylcellulose, hydroxyalkylcellulose (e.g. low substituted hydroxypropyl cellulose), carboxyalkylcellulose (e.g. carboxymethylcellulose), alkali metal salts of carboxyalkylcelluloses (like cross-linked sodium carboxymethylcellulose), carboxyalkyl alkylcellulose, carboxyalkylcellulose esters, cross-linked polyvinyl pyrrolidone (crospovidone), sodium starch glycolate, cellulose ethers, starch, starch derivatives, polyvinyl pyrrolidone, polyrethylene glycol, polyvinyl alcohol, polyvinyl acetate, acrylic polymers, natural, semisynthetic, or synthetic polysaccharides, colloidal silicon dioxide and mixtures thereof. In one embodiment, the gelling agent may comprise cross-linked sodium carboxymethylcellulose.

The amount of the at least one gelling agent may range from about 5 % to about 80 % by weight of the pharmaceutical dosage form. In certain embodiments, the amount of the at least one gelling agent may range from about 10 % to about 70 % by weight, again in certain embodiments, the amount of the gelling agent may range from about 20 % to about 60 % by weight of the pharmaceutical dosage form. In certain embodiments, the amount of the at least one gelling agent may range from about 40 % to about 55 % by weight of the pharmaceutical dosage form.

Inclusion of an alkalizing agent (D) facilitates the gelling kinetics of the gelling agent when the pharmaceutical composition is in contact with a small volume of a suitable solvent. Thus, the presence of the alkalizing agent provides additional abuse deterrent features to the pharmaceutical composition. Non-limiting examples of suitable alkalizing agents include di-and tri-basic phosphate salts (e.g., sodium or potassium phosphate dibasic, or sodium or potassium phosphate tribasic), bicarbonate salts (e.g., sodium or potassium bicarbonate), carbonate salts (e.g., sodium or potassium carbonate), potassium citrate, sodium lactate, calcium acetate and mixtures thereof. In one embodiment, the alkalizing agent may comprise sodium carbonate.

The amount of the at least one alkalizing agent may be up to about 20 % by weight of the pharmaceutical dosage form. In certain embodiments, the amount of the alkalizing agent may be up to about 15 % by weight, again in certain embodiments, the amount of the alkalizing agent may be up to about 10 % by weight of the pharmaceutical dosage form. In certain embodiments, the amount of the at least one alkalizing agent may be up to 5 % by weight of the pharmaceutical dosage form.

In certain embodiments, the pharmaceutical dosage form disclosed herein may further comprise at least one additional pharmaceutically acceptable excipient (E). Non-limiting examples of suitable excipients include binders, fillers, diluents, antioxidants, chelating agents, flavoring agents, coloring agents, taste masking agents, and combinations thereof.

In some embodiments, the pharmaceutical dosage form may further comprise an optional coating. Typically, the coating comprises at least one water-soluble polymer, and the coating does not affect the extended release or abuse deterrent properties of the pharmaceutical dosage form. The coating may provide moisture protection, enhanced appearance, increased mechanical integrity, Improved swallowabillty, Improved taste, and/or masking of odors.

Suitable excipients and coating materials as well as their amounts are well known in the art and disclosed e.g. in "Handbook of Pharmaceutical excipients", 2nd Ed. 25 1994, American Pharmaceutical Association, Washington, Wade A., Weller PJ and in Rowe et. al., "Handbook of Pharmaceutical Exciplents", 7th Ed., London: Pharmaceutical Press, 2012.

In certain embodiments, the substances used for coating of the dosage form include without being limited to carbohydrates, cellulose esters, cellulose ethers, like hydroxylpropylmethylcellulose, starch derivatives, polymers and copolymers comprising polyvinyl pyrrolidone, polyethylene glycol, polyvinyl alcohol, polyvinyl acetate and/or acrylic polymers.

In one embodiment, the dosage form according to the present invention comprises
(A) 5-80 % by weight of at least one API
(B) 5-70 % by weight of at least one PCL
(C) 5-80 % by weight of at least one gelling agent
(D) up to 20 % by weight of at least one alkalizing agent

In another embodiment, the dosage form comprises
(A) 10-70 % by weight of at least one API
(B) 10-60 % by weight of at least one PCL
(C) 10-70 % by weight of at least one gelling agent
(D) up to 15 % by weight of at least one alkalizing agent

In again another embodiment, the dosage form comprises
(A) 10-50 % by weight of at least one API
(B) 20-50 % by weight of at least one PCL
(C) 20-60 % by weight of at least one gelling agent
(D) up to 10 % by weight of at least one alkalizing agent

In again another embodiment, the dosage form comprises
(A) 15-30 % by weight of at least one API
(B) 30-40 % by weight of at least one PCL
(C) 40-55 % by weight of at least one gelling agent
(D) up to 5 % by weight of at least one alkalizing agent

The physical form of the pharmaceutical dosage form may vary. In general, the pharmaceutical composition is a solid dosage form. The solid dosage form may be one of various solid dosage units. Non-limiting examples of suitable solid dosage units include tablets, beats, compacts, pills and dragées. Such dosage units may be prepared using conventional methods known to the skilled person.

In a preferable embodiment, the solid dosage unit may be a tablet. In a preferred embodiment the oral solid dosage form is monolithic.

The solid dosage form is formulated such that the API in the composition is released over an extended period of time. For example, the total amount of the API in the pharmaceutical dosage form may be released over a period of more than 4 hours.

The In vitro dissolution of the API from the solid pharmaceutical dosage form disclosed herein may be measured using a standard USP procedure. For example, dissolution may be measured using an USP approved Type 2 paddle apparatus, at a paddle speed of 50 rpm or 100 rpm, and a constant temperature of 37±0.5°C. The dissolution test may be performed in the presence of 500 ml, 900 ml, or 1000 ml of a suitable dissolution medium (e.g., having a pH from 1.0 to 6.8). Non-limiting examples of suitable dissolution media include water, phosphate buffer (pH 6.8), acetate buffer (pH 4.5), and 0.1 N HCI.

In various embodiments, the in vitro release of the API from the solid pharmaceutical dosage form is such that no more than about 50 %, 60 %, 70 %, 80 %, 90 % or 95 % of the API is released within about 6 hours. In additional embodiments, no more than about 80 % of the API is released within about 6 hours. In still another embodiment, no more than about 50 %, 60 %, 70 %, 80 %, 90 % or 95 % of the API is released within about 8 hours.

The solid pharmaceutical dosage forms according to the present invention demonstrate abuse deterrent properties. The combination of the polycaprolactone, the gelling agent and the optional alkalizing agent imparts sufficient mechanical integrity (i.e., strength, hardness, elasticity, etc.) to the composition such that it is resistant to crushing, grinding, cutting, or pulverizing to form a powder comprising small particles.

The solid dosage form according to the present invention has a breaking strength in any dimensional plane of at least 500 N.

A measure of the mechanical integrity of tablets is their breaking strength, which is the force required to cause them to fail (i.e., break) in a specific dimensional plane. The tablets are generally placed between two platens, one of which moves to apply sufficient force to the tablet to cause fracture. Measurements of breaking strength do not take into account the dimensions or shape of the tablet. Therefore in general, tablets are tested either across the diameter or parallel to the longest axis to facilitate comparability.

Various methods for determining the breaking strength are known to a person skilled in the art, such as Dr. Schleuniger® model 8M (Pharmatron Inc.), Varian model VK200 (Varian Medical Systems Inc.), or Sotax HT1 (Sotax Corp.), for instance. In this analysis, when the integrity of the dosage form is compromised, the instrument will stop compressing and report the force delivered to the dosage form at the breaking strength.

Additionally, since the dosage form comprises gelling polymers, the dosage form forms a viscous mixture or gel when in contact with a small volume of a suitable solvent.

An additional abuse deterrent property Is that the pharmaceutical dosage form, whether whole, flattened, or broken into large particles, forms a viscous mixture or gel when in contact with a small volume of a suitable solvent. The volume of the suitable solvent may range from about 1 ml to about 10 ml. Suitable solvents include e.g. water, vinegar, ethanol, isopropanol, mineral spirits and those that have potentially relevant solvent characteristics (e.g., pH, polarity, protic vs. aprotic), acids such as acetic acid, fruit juice, and mixtures of any of the foregoing.

The resultant gel has a high viscosity that prevents separation of the active ingredient from the viscous gel, provides a visual deterrence to injection abuse, and Inhibits the gelled mixture from being drawn through an injection syringe needle. Consequently, the pharmaceutical dosage forms disclosed herein provide deterrence to abuse by extraction of the API and consequent injection of the extracted mixture.

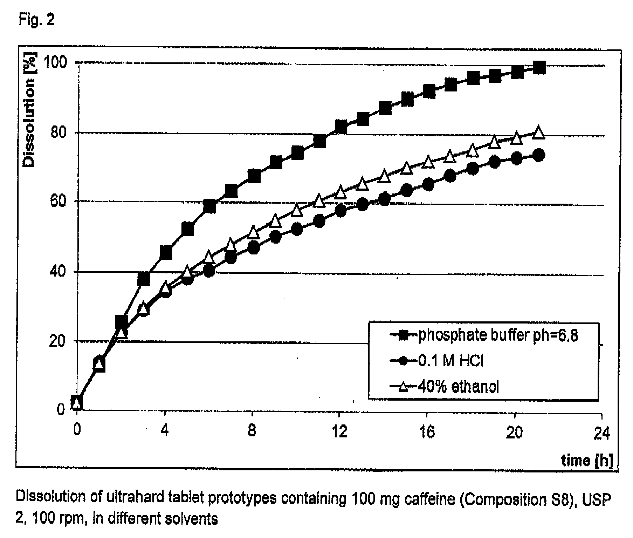

The solid dosage form according to the present invention demonstrates also enhanced anti-dose dumping properties. Often tablets, once misused, are dissolved in alcoholic drinks to achieve dose dumping. When the particle size is reduced by cutting the tablets, the dissolution of the active ingredient is faster due to the increase in surface area. However, when the cut tablets according to the present invention are extracted with other solvents, like 40 % v/v ethanol or 0.1 M HCI, the dissolution is slower compared to a phosphate buffer (Figure 2).

Another aspect of the invention describes an easy process for preparing the solid dosage form according to the present Invention,

The conventional methods to provide dosage forms with high breaking strength known in the art use melt processes like a hot-melt extrusion. The present inventors have surprisingly found that hardening a dosage form comprising at least one PCL, at least one gelling agent and optionally at least one alkalizing agent by tempering results in the ultra-hard dosage forms as provided herein.

Tempering uses significantly lower temperatures than hot-melt extrusion and avoids mechanical stress (e.g. shear-force, pressure) and therefore makes the use of sensitive active ingredients possible. Another advantage inherent to tempering over hot-melt extrusion is the simpler production process, which does not require specialized and therefore expensive equipment.

The solid, abuse deterrent dosage form according to the invention is preferably produced by mixing the at least one API (A), at least one polycaprolactone (B), at least one gelling agent (C), optionally at least one alkalizing agent (D), and optionally at least one excipient (E), and, press-forming the resultant mixture to yield the dosage form, and hardening said dosage form by heating.

Mixing of components (A), (B), (C), optionally (D) and optionally (E) is performed in a mixer known to the person skilled in the art. The mixer may, for example, be a roll mixer, shaking mixer, shear mixer or compulsory mixer. The resultant mixture is preferably formed directly by application of pressure to yield the dosage form according to the invention. The mixture may, for example, be formed into tablets by direct tableting. The compression step Is performed without heating the mixture. After the tablets have been manufactured, they are shortly heated at least to the softening temperature (glass transition temperature, melting temperature; sintering temperature) of component (B) and cooled again. Said hardening step may be performed e.g. In a pan coater, on a tray in a heated cabinet or in a microwave oven. The hardening step may be performed before or after an optional coating step. In a preferred embodiment, the dosage form according to the present invention is prepared by
(a) mixing the API, the PCL, the gelling agent, the optional alkalizing agent and optionally additional excipients to form a mixture,
(b) forming said mixture into a solid form by applying pressure, optionally coating said dosage form, and
(c) hardening said dosage form by heating the dosage form to more than 55°C.

In some embodiments, the forming step is conducted at temperatures lower than 70°C.

In some embodiments, the hardening step is conducted between 55°C and 90°C, 55°C and 80°C, 55°C and 75°C, 55°C and 70°C, 55°C and 65°C; 60°C and 85°C, 60°C and 80°C, 60°C and 75°C, 65°C and 85°C, 65°C and 80°C, 65°C and 75°C, 65°C and 70°C; 70°C and 90°C or 70°C and 85°C, 70°C and 80°C or 70°C and 75°C.

In certain embodiments, the hardening step is conducted by applying dielectric energy supply (e.g., microwave radiation). In some embodiments, the hardening step is conducted by applying dielectric energy supply over 5 s, 10 s, 15 s, 20 s, 30 s, 45 s, 60 s, 90 s or 120 s.

An exemplary process may consist of a direct compression of the blend into tablets, coating the tablets and doing the hardening step by applying heat in a pan coater.

In some embodiments, the hardening step is conducted by applying heat over 10 min, 15 min, 20 min, 30 min, 45 min, 60 min, 90 min or 120 min, in some embodiments, the hardening step is conducted in a pan coater over 10 min, 15 min, 20 min, 30 min, 45 min, 60 min, 90 min or 120 min.

In another aspect of the invention, a solid oral dosage form according to one aspect of the invention, or obtained by a process according to another aspect of the invention, is provided for use in oral, abuse-safe administration of analgesics, like opioid analgesics, anesthetics, sedatives, anxiolytics, hypnotic, anti-adiposity, neuroactive and /or psychoactive drugs.

In yet another aspect of the invention, a solid oral dosage form according to one aspect of the invention, or obtained by a process according to another aspect of the invention, is provided for use In a method of treatment of pain, withdrawal symptoms, neurological or psychiatric disorders, sleep disorders, anxiety and/or obesity.

### EXAMPLES

The present invention is further illustrated by way of the following, non-limiting examples:
Example 1 (method 1): Preparation of hardened tablets
Step 1: Weighing the compounds, sieving through 500 µm mesh size
Step 2: Mixing the compounds in a Turbula mixer for 15 minutes with a rotation speed of 45 rpm
Step 3: Compression of the mixture into tablets on a single punch tablet press.

| | |
|---|---|
| Tooling format: | oblong 15.92 x 8.5 mm, curvature radius 6.92 mm |
| Compression force: | 10 kN |
| Tablet height: | 4-7 mm depending on formulation |

Step 4: Coating the tablets in a pan coater with 2.5 mg/cm² PVA (polyvinyl alcohol)
Step 5: Hardening the tablets in a pan coater for 1 hour at 100°C inlet air temperature. Resulting product temperature: between 60°C and 85°C
Step 6: Cooling the tablets in a pan coater with ambient inlet air temperature for 15 minutes
Example 2 (method 2):
Same as Example 1 but without step 4 (no coating)
Example 3 (method 3):
Steps 1-3 as in Example 1
Step 4: Hardening the tablets on a tray in a heated cabinet at 100°C for 1 hour
Step 5: Cooling the tablets on a tray at room temperature for 30 minutes
Example 4 (method 4):
Same as Example 3 but modified step 4:
   hardening the tablets in a microwave oven at 600W for 20 seconds.

**Table 1: Tablet compositions**

| **Composition no** | **S1** | **S2** | **S3** | **S4** | **S5** | **S6** | **S7** | **S8** |
|---|---|---|---|---|---|---|---|---|
| Method | 2 | 2 | 1 | 2 | 3 | 2 | 4 | 2 |

| **Component** | | | | | | | | |
|---|---|---|---|---|---|---|---|---|
| PCL (MW 50.000) [mg] | 210 | 210 | 210 | 210 | 210 | 210 | 210 | 210 |
| Caffeine (mg) | 100 | 100 | 100 | 100 | 100 | 100 | 100 | 100 |
| Powdered cellulose (mg) | 386 | | | | | | | |
| Microcrystalline cellulose (mg) | | 386 | | | | 193 | 193 | |
| Croscarmellose sodium (mg) | | | 386 | | | | | 380 |
| Mannitol (mg) | | | | 386 | 386 | | | |
| Polyethylene glycol (MW 20.000) [mg] | | | | | | 193 | 193 | |
| Sodium carbonate (mg) | | | | | | | | 6 |
| Magnesium stearate (mg) | 4 | 4 | 4 | 4 | 4 | 4 | 4 | 4 |
| Colloidal silicium dioxide (mg) | 2 | 2 | 2 | 2 | 2 | 2 | 2 | 2 |
| *Tablet weight (sum) [mg]* | *702* | *702* | *702* | *702* | *702* | *702* | *702* | *702* |

| **Composition no** | **S1** | **S2** | **S3** | **S4** | **S5** | **S6** | **S7** | **S8** |
|---|---|---|---|---|---|---|---|---|
| Breaking strength (USP) [N] | >500 | >500 | >500 | >500 | >500 | 395 | 279 | >500 |
| Pulverizable with hammer | no | no | no | no | no | partially | partially | no |

## Claims

1. A solid oral abuse deterrent dosage form comprising:
(A) at least one active pharmaceutical Ingredient susceptible to abuse
(B) at least one polycaprolactone
(C) at least one gelling agent
(D) optionally at least one alkalizing agent,
**characterized in that** said dosage form has a breaking strength of at least 500 N.

2. The solid dosage form according to claim 1, **characterized in that** the dosage form comprises at least one alkalizing agent (D),

3. The solid dosage form according to any of the preceding claim, **characterized in that** said dosage form is an extended release form.

4. The solid dosage form according to any of the preceding claims, **characterized in that** the dosage form is monolithic.

5. The solid dosage form according to any of the preceding claims, **characterized in that** the polycaprolactone (B) has an average molecular mass of about 10.000 to about 250.000 g/mol, preferably about 20.000 to about 150.000 g/mol,

6. The solid dosage form according to any of the preceding claims, **characterized in that** the active pharmaceutical ingredient susceptible to abuse (A) Is selected from the group comprising analgesics, anesthetics, anticonvulsants, sedatives, anxiolytics, hypnotic, anti-adiposity drugs, neuroactive and/or psychoactive drugs, preferably the active pharmaceutical ingredient (A) is selected from opioid analgesics.

7. The solid dosage form according to any of the preceding claims, **characterized in that** the gelling agent (C) is selected from the group comprising cellulose, alkylcellulose, hydraxyalkylcellulose, carboxyalkylcellulose, alkali metal salts of carboxy-alkylcelluloses, like cross-linked sodium carboxymethylcellulose, carboxyalkylalkylcellulose, carboxyalkylcellulose esters, cross-linked polyvinyl pyrrolidone, sodium starch glycolate, cellulose ethers, starch, starch derivatives, polyvinyl pyrrolidone, polyethylene glycol, polyvinyl alcohol, polyvinyl acetate, acrylic polymers, natural, semi-synthetic, or synthetic polysaccharides, colloidal silicon dioxide and mixtures thereof.

8. The solid dosage form according to any of the preceding claims, **characterized in that** the optional alkalizing agent (D) is selected from the group comprising di- and tri-basic phosphate salts, bicarbonate salts, carbonate salts, potassium citrate, sodium lactate, calcium acetate and mixtures thereof.

9. A process for the manufacture of a solid oral abuse deterrent dosage form according to the claims 1 to 8, comprising the steps of:
(a) mixing at least one active pharmaceutical ingredient (A), at least one polycaprolactone (B), at least one gelling agent (C), optionally at least one alkalizing agent (D) and optionally additional excipients (E) to form a mixture,
(b) forming said mixture into a solid form by applying pressure, and
(c) heating said dosage form above 55°C to hardening it,
**characterized in that** said dosage form has a breaking strength of at least 500 N.

10. A solid oral abuse deterrent dosage form according to any one of claims 1 to 8 for use in abuse-safe administration of analgesics, opioid analgesics, anesthetics, sedatives, anxiolytics, hypnotic, anti-obesity, neuroactive and /or psychoactive drugs.

11. A solid oral abuse deterrent dosage form according to any one of claims 1 to 8, for use in a method for treatment of pain, withdrawal symptoms, neurological or psychiatric disorders, sleep disorders, anxiety and/or obesity.

12. A solid oral abuse deterrent dosage form obtainable by a process according to claim 9.

13. Use of the polycaprolactone in the manufacture of a solid oral abuse deterrent dosage forms according to any of the preceding claims.
